# EUROPÄISCHE PATENTSCHRIFT

(11) **EP 1 965 765 B1**
(45) Veröffentlichungstag und Bekanntmachung des Hinweises auf die Patenterteilung: **13.05.2009**
(21) Anmeldenummer: 06777829.0
(22) Anmeldetag: 18.07.2006
(51) Int. Cl.: A61K 9/127

(54) **ZUSAMMENSETZUNG, DIE EINE LIPOSOMALE DISPERSION VON SIO2-NANOPARTIKELN UND EIN ß-1,3-VERKNÜPFTES GLUCANETHERDERIVAT UMFASST**
COMPOSITION CONTAINING A SIO2-NANOPARTICLE LIPOSOMAL DISPERSION AND AN ß-1,3-LINKED GLUCAN-ETHER DERIVATIVE
COMPOSITION COMPRENANT UNE DISPERSION LIPOSOMALE DE NANOPARTICULES DE SIO2 ET UN DERIVE D'ETHER DE GLUCANE A LIAISON ß-1,3

(30) Priorität: 20.07.2005 DE 102005033954
(43) Veröffentlichungstag der Anmeldung: 10.09.2008
(73) Patentinhaber: Ethios S.A., 6901 Lugano (CH)
(72) Erfinder: STEGER, Oskar, 85276 Pfaffenhofen (DE)
(74) Vertreter: Henkel, Feiler & Hänzel
(86) Internationale Anmeldenummer: PCT/EP2006/064369
(87) Internationale Veröffentlichungsnummer: WO 2007/009989

(56) Entgegenhaltungen:
- FR-A1- 2 667 505
- FR-A1- 2 733 760
- US-A1- 2003 199 576
- US-A1- 2004 120 913

## Beschreibung

Die vorliegende Erfindung betrifft eine Zusammensetzung, die eine liposomale Dispersion von SiO₂-Nanopartikeln und ein wasserlösliches β-1,3-verknüpftes Glucanetherderivat umfasst. Insbesondere betrifft die vorliegende Erfindung kosmetische oder pharmazeutische Zusammensetzungen, die die oben angegebenen Bestandteile zusammen mit einem kosmetisch bzw. pharmazeutisch akzeptablen Träger umfassen.

Die Verwendung von Liposomen als Mittel zum Transport von lipophilen wie hydrophilen Wirkstoffen ist in kosmetischen Formulierungen weit verbreitet. Unzählige Literaturstellen sowie auch Patente sind zu diesem Thema auffindbar (vgl. US-A-5 885 260; US-A-6 663 885; DE-A-102 21 863 und DE-A-103 08 852).

In der Natur kommt das zweithäufigste Element Silizium in unzähligen Formen vor, beispielsweise in Form von Bergkristallen, Quarz, Glimmer, Feldspat, Ton usw. Viele dieser Formen finden auch Anwendung zu Heilzwecken wie in Thermalquellen, als Mineralwasser, Schachtelhalm-Tee oder SiO₂-Gele usw. Dem Siliziumoxid werden dabei ganz unterschiedliche Aktivitäten zugeschrieben:
- Stärkung von Bänder und Sehnen
- Straffung vom Bindegewebe
- Behandlung von Osteoporose und Wachstumsstörungen
- Behandlung von Hautfalten, Hautjucken, Hautunreinheiten
- Behandlung von Haarausfall und brüchigen Fingernägel
- Behandlung von Zahnfleisch- und Hautentzündungen
- Behandlung von Cellulite, Neurodermitis und Psoriasis
- Wundheilende Wirkung.

So meinte bereits Louis Pasteur (1822 - 1895): "Kieselsäure ist dazu bestimmt, in Zukunft eine wichtige Rolle bei der Therapie verschiedener Krankheiten zu spielen".

In der Körper- und Gesichtskosmetik wird Siliziumoxid gegen sogenannte Problemhaut respektive Altershaut eingesetzt. Neuerdings findet man auch sogenanntes "bioaktives" Glas auf dem Markt, dem antimikrobielle, entzündungshemmende, Mineralien-anreichernde und oxidationshemmende Aktivitäten nachgesagt werden (Bioactive Glasses: "A Potential New Class of Active Ingredients for Personal Care Products" SÖFW September 2003). Das dabei verwendete Glaspulver besitzt eine Partikelgrösse von typischerweise 5 µm.

Durch die Anwendung von Nanotechnologie eröffnet sich noch eine viel grössere Palette von Funktionalitäten. So sind Produkte mit deutlich kleinerer Partikelgröße herstellbar, beispielsweise ein SiO₂ mit der Grösse von 20nm. SiO₂-Teilchen einer derartigen Partikelgröße weisen eine spezifische Oberfläche nach BET von mindestens etwa 100 m²/g SiO₂ auf.

Leider kranken jedoch die bisher erhältlichen SiO₂-Dispersionen, die Teilchen derartig kleiner Teilchengrößen enthalten, an rascher Gel- und Aggregat-Bildung und verlieren dadurch einige ihrer charakteristischen Eigenschaften.

Die Erfinder der vorliegenden Erfindung haben überraschenderweise festgestellt, dass eine liposomale Dispersion von SiO₂-Nanopartikeln die Stabilität der SiO₂-Nanopartikel deutlich verbessert. Ferner haben sie festgestellt, dass eine Zusammensetzung, die eine solche liposomale Dispersion von SiO₂-Nanopartikeln und ein β-1,3-verknüpftes Glucanetherderivat umfasst, in überraschender Weise ausgezeichnete kosmetische und pharmazeutische Eigenschaften aufweist. Darauf beruht die vorliegende Erfindung.

Gegenstand der vorliegenden Erfindung ist somit eine Zusammensetzung, die eine liposomale Dispersion von SiO₂-Nanopartikeln und ein β-1,3-verknüpftes Glucanetherderivat umfasst.

Bei dem erfindungsgemäß verwendeten β-1,3-verknüpften Glucanetherderivat handelt es sich um ein wasserlösliches Glucanetherderivat, das β-1,6-Verzweigungen aufweisen kann, wobei das unsubstituierte β-1,3-Glucanausgangsmaterial beispielsweise aus der Bäckerhefe isoliert werden kann. In einer bevorzugten Ausführungsform der vorliegenden Erfindung handelt es sich bei dem wasserlöslichen β-1,3-verknüpften Glucanetherderivat um ein wasserlösliches carboxymethyliertes β-1,3-Glucan.

Glucane sind natürliche Polysaccharide, die aus D-Glucopyranosyleinheiten bestehen. Glucane finden sich in der Natur in Hafer, Gerste, Weizen, Mikroorganismen und Pilzen. Die D-Glucopyranosyleinheiten der Glucane, die in Getreidepflanzen vorkommen, sind durch β-1,4- und β-1,3-glycosidische Bindungen verknüpft. Dagegen bestehen die in Hefen und höheren Pilzen vorkommenden Glucane aus einem β-1,3-Gerüst mit gelegentlichen β-1,6-Verzweigungen. Es hat sich gezeigt, dass Glucane mit einem β-1,3-Gerüst ein wirksamer Stimulator des Immunsystems mit der Fähigkeit zur Aktivierung von Makrophagen, Neutrophilen und anderen Zellen, die spezifische Glucanrezeptoren auf ihren Oberflächen tragen, sind. Insbesondere haben Untersuchungen mit wasserlöslichen β-1,3-Glucanen den immunstimulierenden Einfluss derselben und die dadurch ausgelöste Produktion von Cytokinen in immunokompetenten Zellen der Haut, wie Keratinozyten und Langerhansschen Zellen, beschrieben.

Die erfindungsgemäß verwendeten polymeren Glucanetherderivate besitzen neben Monomereinheiten der Formel I auch solche Monomereinheiten der allgemeinen Formel II.

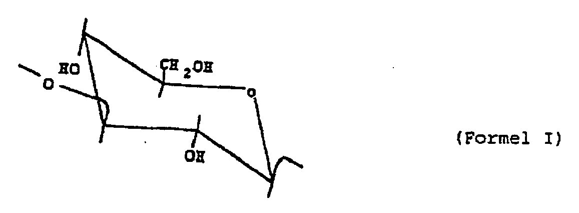

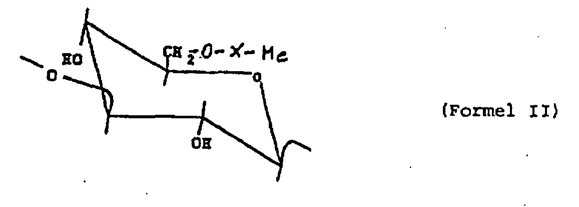

Hierbei sind die Monomereinheiten der Formel 1 mit den Monomereinheiten der Formel II über 1,3-beta-glykosidische Bindungen verknüpft, wobei in Formel II X eine -CH₂-COO-Gruppe, eine -CH₂-CH₂-COO-Gruppe, eine -CH(CH₃)-COO-Gruppe, -CH₂-CH₂-CH₂-COO-Gruppe, -CH₂-CH(CH₃)-CH₂-COO-Gruppe, -CH(CH₂-CH₃)-COO-Gruppe, -CH₂-CH₂-CH₂-CH₂-COO-Gruppe und/oder eine -CH₂-CH₂-SO₃-Gruppe und Me Wasserstoff, ein Alkali- und/oder ein Erdalkalimetall bedeuten.

Die erfindungsgemäß verwendeten polymeren Glucanetherderivate besitzen eine hervorragende Wasserlöslichkeit, wobei abhängig von der Gruppe X und der Gruppe Me in Formel II diese Wasserlöslichkeit um den Faktor 100 bis etwa 500 größer ist als die Wasserlöslichkeit von Glucan. Diese extrem große Wasserlöslichkeit der erfindungsgemäß verwendeten Glucanetherderivate, die absolut gesehen bis zu etwa 80 g pro Liter Wasser beträgt, führt dazu, dass die erfindungsgemäßen Glucanetherderivate ausgezeichnet als Wirkstoffe in pharmazeutischen und/oder kosmetischen Zubereitungen eingesetzt werden können.

Auch lassen sich die erfindungsgemäß verwendeten Glucanetherderivate relativ einfach und sehr schonend herstellen, wie dies nachfolgend noch beschrieben ist. Vorzugsweise besitzen die erfindungsgemäß verwendeten Glucanetherderivate ein Molekulargewicht zwischen 20.000 und 2.000.000, insbesondere zwischen 50.000 und 500.000.

Die gute Wasserlöslichkeit der erfindungsgemäß verwendeten Glucanetherderivate hängt nicht nur von den vorstehend in der Formel II mit X und Me bezeichneten Gruppen, sondern auch von dem Durchschnittssubstitutionsgrad ab, wobei bei den erfindungsgemäß verwendeten Glucanetherderivaten der mittlere Durchschnittssubstitutionsgrad üblicherweise in einem Bereich von 0,25 bis 1,0, üblicherweise 0,5 bis 1,0, vorzugsweise 0,6 bis 0,8, insbesondere bei 0,75 liegt. Ein Substitutionsgrad von 0,25 besagt, dass eine von vier Glucoseeinheiten mit einer in der Formel II mit X und Me bezeichneten Gruppe substituiert ist. Ein erfindungsgemäß bevorzugt verwendetes wasserlösliches Glucanetherderivat (hier auch als CM-Glucan bezeichnet) weist einen durchschnittlichen Substitutionsgrad der Glucoseeinheiten von 0,75 auf, d.h. durchschnittlich 3 von 4 Glucoseeinheiten des β-1,3-Glucans sind mit einer in der Formel II mit X und Me bezeichneten Gruppe substituiert, wobei X für -CH₂-COO- steht und Me Natrium bedeutet (vgl. DE-A-197 10 368; EP-A-1 197 216; EP-A-0 819 703; US-A-6 342 486; US-A-4 833 131 oder US-A-4 454 315).

Insbesondere dann, wenn der mittlere Durchschnittssubstitutionsgrad 0,75 beträgt und wenn in der Formeln II X eine -CH₂-COO-Gruppe und Me Natrium darstellt, besitzt ein derartiges Carboxymethylglucan-Natrium eine ausgezeichnete Wasserlöslichkeit, die im Bereich von etwa 40 g/l Wasser liegt. Von daher wird dieses spezielle Glucanetherderivat, das aus Einheiten der nachfolgenden Formel IV besteht, bevorzugt zur Herstellung von topisch applizierbaren pharmazeutischen und/oder kosmetischen Zusammensetzungen, wie diese nachfolgend noch im Detail beschrieben sind, eingesetzt.

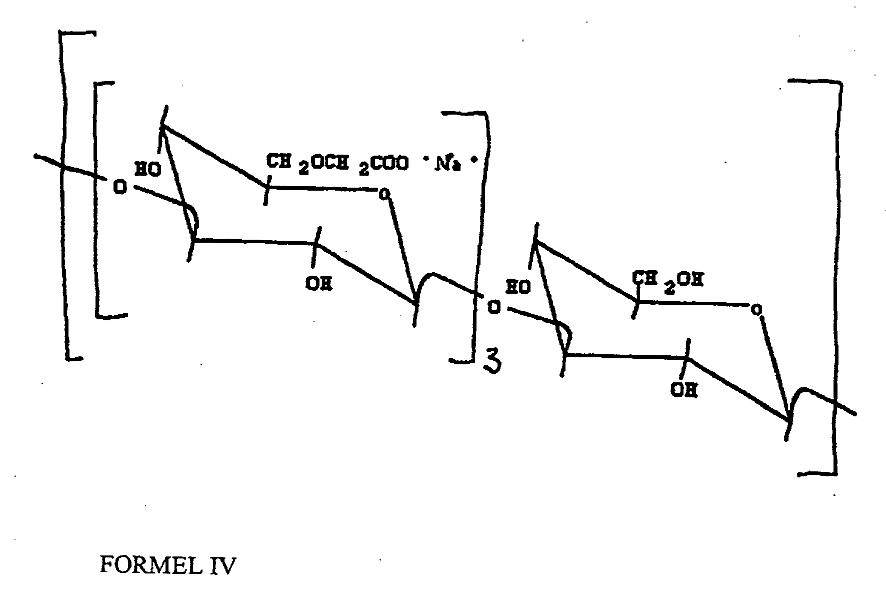

Grundsätzlich können bei den erfindungsgemäß verwendeten Glucanetherderivaten der in der Formeln II mit Me gekennzeichnete Rest Wasserstoff, ein Alkalimetall und/oder ein Erdalkalimetall bedeuten. Besonders dann, wenn in der Formel II Me für ein Alkalimetall, vorzugsweise für Natrium, steht, weist ein derartiges konkretes Glucanetherderivat eine hohe Hautverträglichkeit auf, so dass dieses Glucanetherderivat selbst bei empfindlichen Anwendern keine Hautreizungen verursacht.

Dies trifft auch auf solche Glucanetherderivate zu, bei denen in der vorstehend wiedergegebenen Formel II X für eine -CH₂COO-Gruppe und/oder für eine -CH₂-CH₂-SO₃-Gruppe steht, wobei festgestellt werden konnte, dass diese konkreten Glucanetherderivate neben einer guten Wasserlöslichkeit und einer hohen Hautverträglichkeit des weiteren ausgezeichnete Wirkstoffeigenschaften besitzen, so dass derartige Glucanetherderivate bevorzugt in topisch applizierbaren pharmazeutischen und/oder kosmetischen Zubereitungen eingesetzt werden, die zur Prophylaxe und/oder Therapie von Hauterkrankungen, Hautverletzungen, Hautreizungen, sowie Hautalterung verwendet werden.

Nachfolgend wird ein Verfahren beschrieben, mit dem die vorstehend genannten Glucanetherderivate besonders einfach herstellbar sind.

Das Verfahren zur Herstellung von polymeren Glucanetherderivaten, die neben Monomereinheiten der Formel I auch solche Monomereinheiten der allgemeinen Formel II aufweisen, 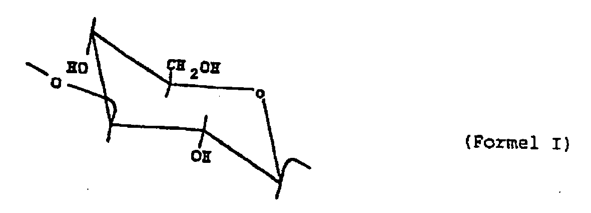 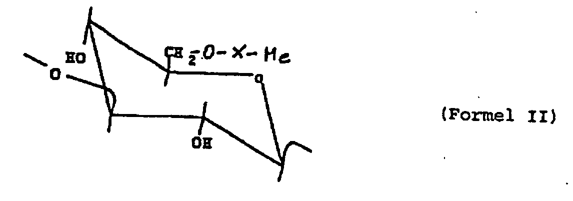 wobei die Monomereinheiten der Formel I mit den Monomereinheiten der Formel II über eine 1,3-beta-glykosidische Bindung verknüpft sind und wobei in Formel II X eine -CH₂-COO-Gruppe, eine -CH₂-CH₂-COO-Gruppe, eine -CH(CH₃)-COO-Gruppe, -CH₂-CH₂-CH₂-COO-Gruppe, -CH₂-CH(CH₃)-CH₂-COO-Gruppe, -CH(CH₂-CH₃)-COO-Gruppe, -CH₂-CH₂-CH₂-CH₂-COO-Gruppe und/oder eine -CH₂-CH₂-SO₃-Gruppe und Me Wasserstoff, ein Alkali- und/oder ein Erdalkalimetall bedeuten, sieht vor, dass
a) gereinigtes und weitestgehend lipidfreies Glucan in einem niedrigen C₁-C₄-Alkohol, vorzugsweise Isopropanol, unter Zusatz einer alkalischen Lösung behandelt wird,
b) hiernach ein Teil der alkalischen Lösung entfernt wird,
c) die dabei anfallende Suspension auf eine Temperatur zwischen 40 °C und 60 °C erwärmt und anschließend bei dieser Temperatur mit einer wässrigen Lösung eines Salzes einer Halogencarbonsäure oder einer Halogensulfonsäure der nachfolgenden Formeln III 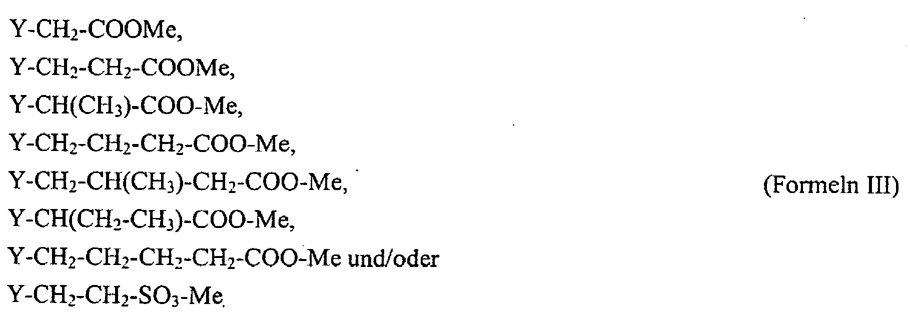 umgesetzt wird, wobei in den Formeln III Y ein Halogen und Me ein Alkali- und/oder Erdalkalimetall bedeuten,
d) nach einer Reaktionszeit von einer Stunde bis vier Stunden das so gebildete Glucanetherderivat gewaschen und
e) das gewaschene Glucanetherderivat schonend getrocknet wird.

Das Verfahren weist zunächst den entscheidenden Vorteil auf, dass es relativ einfach und mit geringem apparativem Aufwand durchführbar ist. Des weiteren konnte festgestellt werden, dass sich nach dem zuvor beschriebenen Verfahren die in den Formeln I und II wiedergegebenen polymeren Glucanetherderivate besonders schonend und mit hoher Reinheit herstellen lassen, so dass dementsprechend die erfindungsgemäß verwendeten Glucanetherderivate schnell verfügbar sind. Auch treten bei dem beschriebenen Verfahren keine unerwünschten Nebenreaktionen, so z.B. eine unerwünschte Spaltung der 1,3-beta-glykosidischen Bindung auf, was dazu führt, dass die erfindungsgemäß verwendeten polymeren Glucanetherderivate besonders reproduzierbar herstellbar sind und keine unerwünschten Nebenprodukte beinhalten. Dementsprechend sind die erfindungsgemäß verwendeten Glucanetherderivate hervorragend für pharmazeutische und/oder kosmetische Zubereitungen einsetzbar, wie dies nachfolgend noch näher beschrieben ist.

Grundsätzlich kann bei dem beschriebenen Verfahren die vorstehend in der Stufe a) wiedergegebene Behandlung des gereinigten und weitestgehend lipidfreien Glucans unter Zusatz einer alkalischen Lösung bei jeder beliebigen Temperatur durchgeführt werden, wobei es jedoch besonders vorteilhaft ist, wenn diese Behandlung des gereinigten und weitestgehend lipidfreien Glucans bei Raumtemperatur, d.h. in einem Temperaturbereich zwischen etwa 16 °C und etwa 22 °C, durchgeführt wird. Dies hat den entsprechenden Vorteil, dass unerwünschte Nebenreaktionen schon in der ersten Reaktionsstufe a) des beschriebenen Herstellungsverfahrens vermieden werden, so zum Beispiel eine unerwünschte Hydrolyse der 1,3-beta-glykosidischen Bindung, so dass dementsprechend die Reproduzierbarkeit und die Ausbeute des beschriebenen Verfahrens noch weiter verbessert wird.

Wird bei dem beschriebenen Verfahren der vorstehend unter Punkt a) wiedergegebene Reaktionsschritt bei Raumtemperatur ausgeführt, so variiert man abhängig von der zu erzielenden Ausbeute des jeweils zu synthetisierenden polymeren Glucanetherderivats und dessen Reinheit die Reaktionszeit zwischen etwa 10 Stunden und 25 Stunden, vorzugsweise zwischen etwa 12 Stunden und 20 Stunden.

Vorstehend ist bei dem beschriebenen Verfahren bei dem zuvor erwähnten ersten Reaktionsschritt a) beschrieben, dass das gereinigte und weitestgehend lipidfreie Glucan in einem niedrigen Alkohol suspendiert wird. Hierfür wird vorzugsweise ein C₁-C₄-Alkohol, insbesondere Isopropanol, angewendet, wobei das Massenverhältnis von Glucan zu dem C₁-C₄-Alkohol bzw. zu dem Isopropanol zwischen 1:10 his 1:30, vorzugsweise zwischen 1:15 his 1:25, variiert. Ein derartiges konkretes Massenverhältnis bewirkt unter anderem, dass bei dieser Ausführungsvariante des beschriebenen Verfahrens eine quantitative Umsetzung der Glucansuspension mit der vorzugsweise als wässrige Natronlauge vorliegenden alkalischen Lösung erfolgt, was wiederum einen Einfluß auf die Reinheit des so hergestellten erfindungsgemäß verwendeten Glucanetherderivats sowie auf die Wirtschaftlichkeit des beschriebenen Verfahrens hat.

Um bei dem beschriebenen Verfahren im eingangs wiedergegebenen Reaktionsschritt a) eine unerwünschte Nebenreaktion, so zum Beispiel die Spaltung der im Glucan enthaltenen 1,3-βglykosidischen Bindung, zu unterdrücken, sieht eine Weiterbildung des beschriebenen Verfahrens vor, dass außer und/oder zusätzlich zu dem zuvor erwähnten Temperaturbereich sowie den zuvor beschriebenen Reaktionszeiten als alkalische Lösung eine 20 gew.%-ige bis 35 gew.%-ige wässrige Natronlauge in einem Massenverhältnis von Glucan zu Natronlauge zwischen 1:1,5 und 1:3 verwendet wird.

Bei einer anderen Weiterbildung des beschriebenen Verfahrens wird, wie vorstehend bei dem beschriebenen Verfahren im Reaktionsschritt b) beschrieben, nach dem Entfernen des Teiles der alkalischen Lösung und vor dem Erwärmen der Suspension eine weitere Menge eines niedrigen Alkohols, vorzugsweise des C₁-C₄-Alkohols und insbesondere des Isopropanols, dem Reaktionsgemisch zugesetzt, so dass anschließend dann die um die entsprechende Alkoholmenge ergänzte Suspension auf eine Temperatur zwischen 40 °C und 60 °C erwärmt wird, wie dies bereits vorstehend beim beschriebenen Verfahren unter dem Reaktionsschritt c) beschrieben ist. Hierbei dient diese Maßnahme ebenfalls dazu, einerseits die Hydrolyse zu unterdrücken und andererseits eine gezielte Reaktion in der Position 6 herbeizuführen, so dass dementsprechend durch Anwendung dieser Verfahrensvariante hochreine Glucanetherderivate hergestellt werden können.

Grundsätzlich besteht bei der zuvor beschriebenen Weiterbildung des beschriebenen Verfahrens die Möglichkeit, dass die zugesetzte Menge des niedrigen Alkohols nicht der Menge der teilweise entfernten alkalischen Lösung entspricht, wobei jedoch festgestellt werden konnte, dass besonders gute Ausbeuten dann bei dem beschriebenen Verfahren erzielbar sind, wenn die zugesetzte Menge des niedrigen Alkohols der Menge der teilweise entfernten alkalischen Lösung entspricht.

Bei dem beschriebenen Verfahren wird vorstehend unter Punkt d) vorgesehen, dass das nach dem beschriebenen Verfahren hergestellte Glucanetherderivat nach Ablauf einer Reaktionszeit zwischen 1 Stunde und 4 Stunden gewaschen wird. Grundsätzlich kann zum Waschen des nach dem beschriebenen Verfahren hergestellten Glucanetherderivats jedes geeignete organische Lösemittel verwendet werden, in dem das gebildete Glucanetherderivat nicht oder nur bedingt löslich ist. Eine besonders geeignete Weiterbildung des beschriebenen Verfahrens schlägt vor, dass zum Waschen des Glucanetherderivats der Zusatz einer Alkohol/Wasser-Mischung erfolgt, wobei insbesondere Isopropanol-/Wasser-Mischungeri in einem Volumenverhältnis von Isopropanol zu Wasser von 1:1 bis 1,5:1 besonders bevorzugt sind.

Um bei dem beschriebenen Verfahren nach Abschluss der vorstehend unter Punkt d) angegebenen Reaktionszeit ein Abbruch der Reaktion sicher und reproduzierbar herbeizuführen, sieht eine andere Weiterbildung des beschriebenen Verfahrens vor, dass das Reaktionsgemisch nach Ablauf der Reaktionszeit von 1 Stunde bis zu 4 Stunden durch Zusatz einer Säure, vorzugsweise durch Zusatz von konzentrierter Salzsäure, auf einen pH-Wert zwischen 6,8 und 7,5 neutralisiert wird.

Um bei dem beschriebenen Verfahren das hiernach hergestellte gewaschene und/oder neutralisierte Glucanetherderivat aus dem Reaktionsgemisch zu isolieren, wird bevorzugt das so hergestellte und im wässrigen System lösliche erfindungsgemäß verwendete Glucanetherderivat durch Zusatz eines niedrigen Alkohols, vorzugsweise durch Zusatz von Isopropanol, aus der wässrigen Reaktionsmischung ausgefällt. Durch diese Variante des beschriebenen Verfahrens kann dass so hergestellte Glucanetherderivat nahezu quantitativ aus dem Reaktionsgemisch entfernt werden, wobei ein derartig isoliertes Glucanetherderivat dann noch eine hohe Reinheit aufweist.

Bezüglich der sich an die Isolierung anschließende Trocknung ist festzuhalten, dass diese Trocknung des nach dem Verfahrens hergestellten Glucanetherderivats vorzugsweise schonend zu erfolgen hat. Hier hat sich gezeigt, dass eine Vakuumtrocknung in einem Temperaturbereich zwischen 40 °C und 80 °C ganz besonders diesen Anforderungen genügt, so dass eine derartige Trocknung bei dem beschriebenen Verfahren bevorzugt wird.

Bei den erfindungsgemäß zur Herstellung der liposomalen Dispersion von SiO₂-Nanopartikeln verwendeten SiO₂-Nanopartikeln handelt es sich allgemein um SiO₂-artikel einer mittleren Teilchengröße von etwa 1 bis 1000 nm, üblicherweise 1 bis 500 nm, vorzugsweise 1 bis 100 nm, insbesondere 1 bis 40 nm, speziell 2 bis 20 nm.

Die Herstellung der erfindungsgemäß verwendeten liposomalen Dispersion von SiO₂-Nanopartikeln erfolgt üblicherweise nach dem folgenden Verfahren:

Zunächst wird ein Phospholipid, üblicherweise ein Lecithin, in einer wässrigen Lösung in einer Konzentration von üblicherweise 1 bis 20 Gew.-%, vorzugsweise 2 bis 15 Gew.-% und vorzugsweise 3 bis 10 Gew.-% gelöst. Die wässrige Lösung kann ferner einen Alkohol, beispielsweise Methanol, Ethanol, Propanol, Ethylenglykol oder Glycerin, vorzugsweise Glycerin, in einer Konzentration von 10 bis 99 Gew.-%, vorzugsweise 30 bis 98 Gew.-%, vorzugsweise 50 bis 98 Gew.-%, enthalten. Danach werden die SiO₂-Nanopartikel in einer Pufferlösung üblicherweise in einer Konzentration von 2 bis 70 Gew.-%, vorzugsweise 10 bis 60 Gew.-%, insbesondere 20 bis 50 Gew.-% suspendiert. Anschließend wird die phospholipidhaltige Lösung unter Rühren in die wässrige SiO₂-Suspension eingetragen. Das Gemisch wird anschließend einem Hochdruckhomogenisator zugeführt oder einer Ultraschallbehandlung unterzogen. Üblicherweise wird das Gemisch mehrmals durch den Hochdruckhomogenisator geführt, um eine liposomale Dispersion geeigneter Partikelgrösse, beispielsweise von Liposomen einer Größe von <1000 nm, üblicherweise <100 nm, vorzugsweise <70nm, insbesondere <50 nm zu erreichen. So ist zum Erreichen einer Liposomengröße von <50 nm beispielsweise ein mindestens zweimaliges Führen des Gemisches durch den Hochdruckhomogenisator erforderlich. Der Hochdruckhomogenisator umfasst dabei eine Mikrofluidisiervorrichtung mit Mikrokanälen in einem Keramikblock, in denen das Gemisch aus Phospholipid und SiO₂-Nanopartikeln unter dem Einfluß hoher Scherkräfte bei Fließgeschwindigkeiten von bis zu 500 m/s Drücken von bis zu 1200 bar ausgesetzt wird.

In einer bevorzugten Ausführungsform der vorliegenden Erfindung liegt die Teilchengröße der liposomalen Dispersion in einem Bereich von 20 bis 400 nm, insbesondere in einem Bereich von 30 bis 100 nm.

Gegenstand eines weiteren Aspekts der vorliegenden Erfindung ist eine pharmazeutische Zubereitung, die eine liposomale Dispersion von SiO₂-Nanopartikeln, ein β-1,3-verknüpftes Glucanetherderivat und einen pharmazeutisch akzeptablen Träger umfasst.

Ein weiterer Aspekt der Erfindung ist die Verwendung einer liposomalen Dispersion von SiO₂-Nanopartikeln und eines β-1,3-verknüpften Glucanetherderivats entsprechend den vorhergehenden Definitionen zur Herstellung eines Medikaments zur Behandlung eines Zustands oder einer Erkrankung, wie Krebs, beispielsweise Leukämie oder Darmkrebs, lokale Invasion von Tumoren in benachbarte Bereiche, Metastasenausbreitung von Tumorzellen von primären zu sekundären Stellen, Asthma, Osteoporose, einer degenerativen Erkrankung des Bindegewebes und/oder der Gefäße, eines Verbesserung der Immunabwehr, von Herpes, Aphthen, Entzündungen, beispielsweise der Mundschleimhaut, des Zahnfleisches und/oder des Rachens, Parodontose, Warzen, Akne, Verbrennungen, Schürfwunden, Sonnenbrand, Stichen, Ekzemen, Haarausfall, Psoriasis, Fibrose und Gewebezerstörung bei Arthritis.

Für eine medizinische Verwendung gemäß dieser Erfindung kann die zum Erreichen einer therapeutischen Wirkung erforderliche Menge der liposomalen Dispersion von SiO₂-Nanopartikeln und des β-1,3-verknüpften Glucanetherderivats natürlich sowohl mit dem Verabreichungsweg, dem in Behandlung stehenden Säuger als auch der speziellen Störung oder entsprechenden Erkrankung variieren. Eine geeignete Dosis einer liposomalen Dispersion der SiO₂-Nanopartikel sowie des β-1,3-verknüpften Glucanetherderivats für einen Säuger, der an einer der im Vorhergehenden beschriebenen Erkrankungen leidet oder wahrscheinlich daran leidet, beträgt 0,1 µg bis 500 mg der jeweiligen Komponente pro kg Körpergewicht. Im Fall der systemischen Verabreichung kann die Dosis im Bereich von 0,5 bis 400 mg der jeweiligen Komponente pro kg Körpergewicht sein, wobei die bevorzugte Dosierung 0,5 bis 50 mg/kg Körpergewicht des Säugers beträgt, die zwei- bis dreimal täglich verabreicht wird. Im Falle der topischen Verabreichung, beispielsweise auf Haut oder Auge, kann eine geeignete Dosis im Bereich von 0,1 ng bis 100 µg der jeweiligen Komponente pro kg, typischerweise etwa 0,1 bis 50 µg/kg betragen.

Es ist klar, dass ein Arzt üblicher Erfahrung die zur Verhinderung oder zum Stoppen des Fortschreitens der Erkrankung, für die die Behandlung verabreicht wird, wirksame Menge ohne weiteres bestimmen und verschreiben kann. Bei diesem Vorgehen kann der Arzt zunächst relativ niedrige Dosen verwenden, und anschließend die Dosis erhöhen, bis ein maximales Ansprechen erhalten wird.

Üblicherweise enthält die pharmazeutische Formulierung, die eine liposomale Dispersion von SiO₂-Nanopartikeln und ein β-1,3-verknüpftes Glucanetherderivat umfasst, einen phannakologisch akzeptablen Träger hierfür.

Die Formulierungen sowohl zur humanmedizinischen Verwendung umfassen die Wirkstoffkombination in Verbindung mit einem pharmazeutisch akzeptablen Träger hierfür und optional einen anderen therapeutischen Bestandteil bzw. andere therapeutische Bestandteile. Der Träger bzw. die Träger müssen "akzeptabel" in einem Sinne sein, dass sie mit den anderen Bestandteilen der Formulierungen kompatibel und für den Empfänger derselben nicht schädlich sind.

Die Formulierungen umfassen Formulierungen in einer Form, die zur oralen, pulmonalen, ophthalmologischen, rektalen, parenteralen (einschließlich der subkutanen, intramuskulären und intravenösen), intraartikulären, topischen, nasalen oder bukkalen Verabreichung geeignet ist. Derartige Formulierungen sollen einschlägig bekannte Formulierungen mit Langzeitwirkung umfassen.

Die Formulierungen können günstigerweise in Einheitsdosisform präsentiert werden und nach einem der auf dem Gebiet der Pharmazie bekannten Verfahren hergestellt werden. Alle Verfahren können die Stufe des In-Verbindung-Bringens des Wirkstoffs mit dem Träger, der einen oder mehrere Nebenbestandteile darstellt, umfassen. Im allgemeinen werden die Formulierungen durch gleichförmiges und inniges In-Verbindung-Bringen des Wirkstoffs mit einem flüssigen Träger oder einem feinzerteilten festen Träger oder beiden und, falls notwendig, anschließendes Formen des Produkts zur gewünschten Formulierung hergestellt.

Zur oralen Verabreichung geeignete Formulierungen der vorliegenden Erfindung können in der Form diskreter Einheiten, wie Kapseln, Kachets, Tabletten oder Pastillen, die jeweils eine vorbestimmte Menge des Wirkstoffs enthalten; in der Form eines Pulvers oder Granulats; in der Form einer Lösung oder einer Suspension in einer wässrigen Flüssigkeit oder nichtwässrigen Flüssigkeit; oder in der Form einer Öl-in-Wasser-Emulsion oder einer Wasser-in-Öl-Emulsion sein. Der Wirkstoff kann auch in Form eines Bolus, einer Latwerge oder einer Paste sein.

Zubereitungen fester Form umfassen Pulver, Tabletten, dispergierbare Granulatkörnchen, Kapseln, Kachets und Suppositorien. Ein fester Träger kann eine oder mehrere Substanzen sein, die auch als Verdünnungsmittel, Aromatisierungsmittel, Solubilisierungsmittel, Gleitmittel, Suspendiermittel, Bindemittel oder den Tablettenzerfall fördernde Mittel fungieren können; er kann auch ein Einkapselungsmaterial sein. In Pulvern ist der Träger ein feinzerteilter Feststoff, der im Gemisch mit der Wirkstoffkombination vorliegt. Bei der Tablette wird die Wirkstoffkombination mit einem Träger mit den notwendigen Bindeeigenschaften in geeigneten Anteilen gemischt und in der gewünschten Form und Größe kompaktiert. Die Pulver und Tabletten enthalten vorzugsweise 0,1 bis etwa 70 % der jeweiligen Wirkstoffkomponente. Geeignete feste Träger sind Magnesiumcarbonat, Magnesiumstearat, Talkum, Zucker, Lactose, Pektin, Dextrin, Stärke, Gelatine, Tragant, Methylcellulose, Natriumcarboxymethylcellulose, ein niedrigschmelzendes Wachs, Kakaobutter und dergleichen. Der Ausdruck "Zubereitung" soll die Formulierung der aktiven Verbindung mit Einkapselungsmaterial als Träger, wobei eine Kapsel erhalten wird, in der die aktiven Komponenten (mit oder ohne andere Träger) von Träger umgeben ist, der daher mit diesen in Verbindung steht, umfassen. In ähnlicher Weise werden Kachets umfasst. Tabletten, Pulver, Kachets, transdermale und transmucosale Systeme und Kapseln können als zur oralen Verabreichung geeignete feste Dosierungsformen verwendet werden.

Zubereitungen flüssiger Form umfassen Lösungen, Suspensionen und Emulsionen. Als Beispiel können Wasser- oder Wasser-Propylenglykollösungen zur parenteralen Injektion genannt werden. Flüssige Zubereitungen können auch in Lösung in einer wässrigen Polyethylenglykollösung formuliert werden. Zur oralen Verwendung geeignete wässrige Lösungen können durch Auflösen der aktiven Komponente in Wasser und die Zugabe geeigneter Farbmittel, Aromastoffe, Stabilisierungs- und Dickungsmittel nach Wunsch hergestellt werden. Zur oralen Verwendung geeignete wässrige Suspensionen können durch Dispergieren der feinzerteilten aktiven Komponente in Wasser mit viskosem Material, d.h. natürlichen oder synthetischen Gummis, Harzen, Methylcellulose, Natriumcarboxymethylcellulose und anderen bekannten Suspendiermitteln hergestellt werden. Vorzugsweise liegt die pharmazeutische Zubereitung in Einheitsdosisform vor. In dieser Form ist die Zubereitung in Einheitsdosen unterteilt, die geeignete Mengen der aktiven Komponenten enthalten. Die Einheitsdosisform kann eine abgepackte Zubereitung, wobei die Packung diskrete Mengen der Zubereitung enthält, beispielsweise abgepackte Tabletten, Kapseln und Pulver in Phiolen oder Ampullen, sein. Die Einheitsdosisform kann ebenfalls eine Kapsel, ein Kachet oder eine Tablette selbst sein oder es kann die entsprechende Zahl von diesen in abgepackter Form sein.

Gegenstand eines weiteren Aspekts der vorliegenden Erfindung ist eine kosmetische Zubereitung, die eine liposomale Dispersion von SiO₂ einer Partikelgröße im Bereich von 2 bis 1000 nm, ein β-1,3-verknüpftes Glucanetherderivat und einen kosmetisch akzeptablen Träger enthält.

Ohne an irgendeine Theorie gebunden werden zu wollen, gehen die Erfinder der vorliegenden Erfindung gegenwärtig davon aus, dass durch Zugabe eines β-1,3-verknüpften Glucanetherderivats zu einer liposomalen Dispersion von SiO₂-Nanopartikeln eine Zusammensetzung erhalten wird, die synergistische Aktivitäten zeigt. Die hohe Wasserbindungsfähigkeit beider Wirkstoffe (SiO₂-Nanopartikel & β-1,3-verknüpftes Glucanetherderivat) bewirkt eine signifikante Stabilisierung und Regelung des Feuchtigkeitsgehalts der Haut. Die Tripelhelixstruktur des β-1,3-verknüpften Glucanetherderivats bringt ein sehr hohes Molekulargewicht von bis zu 2.000.000 Dalton mit sich und ist deshalb optimal geeignet zur Ausbildung einer Barrierefunktion auf der Haut. Gleichzeitig können die sehr kleinen liposomal verkapselten SiO₂-Nanopartikel tief in die Haut eindringen, auf diese Weise steht das SiO₂ der Haut länger zur Verfügung.

Die erfindungsgemäße kosmetische Zubereitung eignet sich zur adjuvanten Behandlung von Unannehmlichkeiten im Zusammenhang mit kranker Haut etwa Hautrissen, Verletzungen und Entzündungsreaktionen wie Schuppenflechte, Ekzeme oder Sonnenerythemen. Andererseits werden auch die Phänomene des Alterungsprozesses als Folge einer allgemeinen Dehydratation und Degeneration präventiv und nachhaltig verbessert. Die kosmetische Zusammensetzung der vorliegenden Erfindung kann beispielsweise die Form eines Puders, Kuchens, Stifts, Stäbchens, einer Salbe, einer Flüssigkeit und dergleichen aufweisen. Speziell können Gesichtskosmetika, wie eine Lotion, eine Emulsion (oder milchige Lotion), eine Creme, ein Makeup-Kosmetikum, wie eine Grundierung, ein Lippenstift, Lidschatten, Rouge, Lidstrich, Nagellack, Maskara, Haarkosmetika, wie eine Haarbehandlung, eine Haarflüssigkeit, Frisierlotionen genannt werden.

Typischerweise umfasst die pharmazeutische und/oder kosmetische Zubereitung gemäß der vorliegenden Erfindung die liposomale Dispersion von SiO₂-Nanopartikeln in einer Menge von 0,1 - 60 %, vorzugsweise 0.5 - 20 %, und das β-1,3-verknüpfte Glucanetherderivat in einer Menge von 0,01 - 0,5 Gew.-%, vorzugsweise 0,02 - 0,2 Gew.-%, jeweils bezogen auf das Gewicht der gesamten kosmetischen Zubereitung.

Es ist anzumerken, dass die kosmetische Zusammensetzung der vorliegenden Erfindung zusätzlich zu den im vorhergehenden genannten wesentlichen Komponenten verschiedene Arten von Komponenten, die normalerweise in Kosmetika verwendet werden, in einem Bereich, der die Wirkung der vorliegenden Erfindung nicht beeinträchtigt, enthalten kann.

Beispielsweise können feste und halbfeste Öle, wie Vaseline, Lanolin, Ceresin, Camauba-Wachs, Candelilla-Wachs, höhere Fettsäuren, ein höherer Alkohol; flüssige Öle, wie Squalan, Paraffinöl, Esteröle, Triglyceride; Öle, wie Siliconöl; Feuchthaltemittel, wie Natriumhyaluronat, Glycerin; oberflächenaktive Mittel, wie kationische oberflächenaktive Mittel, nichtionische oberflächenaktive Mittel; Antioxidationsmittel, UV-Absorptionsmittel; UV-Blocker; Farbstoffe; Pigmente; Konservierungsmittel; Duftstoffe; Aktivatoren und dergleichen in geeigneter Weise einformuliert werden.

### BEISPIELE

Die vorliegende Erfindung wird nun durch die folgenden Beispiele weiter erläutert. Es ist anzumerken, dass die Mengen, falls nicht anders angegeben, Gewichtsprozentangaben sind.

### Beispiel 1

### Herstellung einer liposomalen Dispersion von SiO₂-Nanopartikeln

Zur Herstellung einer liposomalen Dispersion von SiO₂-Nanopartikeln werden 50g Lecithin in 500g Glycerin gelöst und das Gemisch über nacht stehen gelassen. Anschließend werden zu der Lecithinlösung in Glycerin 100g einer wässrigen SiO₂-Nanopartikelsuspension mit einem SiO₂-Gehalt von 40 Gew.-% und mit einer durchschnittlichen Partikelgröße von 15 nm gegeben. Danach werden zu dieser Mischung noch 350g Wasser gegeben und das Ganze kräftig gerührt. Diese Mischung wird dann 3 mal bei einem Druck von 1200 bar durch einen Microfluidizer MT1 gepumpt. Die liposomale SiO₂-Nanopartikeldispersion weist eine durchschnittliche liposomale Partikelgrösse von 70 nm auf.

### Beispiel 2

### Herstellung von β-1,3-verknüpften Glucanetherderivats

1 kg Glucan werden in 201 Isopropanol unter Rühren in der Kälte suspendiert und mit 2 kg 30 Gew.-% wässriger Natriumhydroxidlösung während 16 h bei Raumtemperatur (16-22 °C) behandelt.

Nach Abschluß dieser alkalischen Behandlung wird nach einer kurzen Verweilzeit von etwa 20 min. der über der Suspension stehende Überstand von 8 l des wässrigen alkalischen Isopropanols abdekantiert. Anschließend erfolgt die Zugabe von 8 l Isopropanol.

Unter Rühren wird die Suspension dann auf 60 °C erwärmt. Nach Erreichen der Endtemperatur werden 1,5 kg des Natriumsalzes der Chloressigsäure in einer Lösung in 1,5 kg Wasser zugesetzt.

Nach einer Gesamtreaktionszeit von 2,5 h bei 60 °C wird das Reaktionsprodukt sedimentiert und die darüber befindliche Flüssigkeit abdekantiert. Anschließend werden 4 l einer wässrigen Isopropanollösung (v:v, 50%:50%) zugegeben.

Nachdem das Reaktionsprodukt gewaschen ist, wird das Natriumsalz des Carboxymethylglucans in 20 I Wasser gelöst. Danach werden zu der Lösung soviel 10 N Salzsäure zugegeben, dass der pH-Wert der Lösung auf einen Wert von 7 eingestellt wird.

Aus einer neutralisierten wässrigen Lösung wird das Natriumsalz des Carboxymethylglucans durch Zusatz von 40 1 Isopropanol ausgefällt. Nach Abtrennung des ausgefällten Natriumsalzes des Carboxymethylglucans wird dieses im Vakuumtrockenschrank bei 60 °C getrocknet. Die Ausbeute an Natriumsalz des Carboxymethylglucans beträgt 1,3 kg. Das Natriumsalz des Carboxymethylglucans zeigt bei einer Analyse des Produkts einen durchschnittlichen Substitutionsgrad von 0,75 ± 0,1.

### Beispiel 3 und Vergleichsbeispiel 1

Herstellung einer erfindungsgemäßen kosmetischen Zubereitung in Form einer Emulsion:
Emulsion:
   Zusammensetzung W1:
      50g Pentylengykol
      30g Glycerin
      30g des oben in Beispiel 2 hergestellten Carboxymethylglucans in Form einer 2% wässrigen Lösung
      20g der in Beispiel 1 erhaltenen liposomalen SiO₂-Nanopartikeldispersion
      20g Harnstoff
      10g Panthenol
      10g Carbopolgel (Alkylacrylat-Copolymer)
      720g Wasser

Die oben genannten Bestandteile werden zusammengegeben und das Gemisch gründlich gerührt.
Zusammensetzung O1:
   100g Mandelöl
   10g Phenoxyethanol

Die oben genannten Bestandteile werden zusammengegeben und gründlich gerührt.

Anschließend wird die Mischung O1 zur Mischung W1 gegeben und mit einem Stabhomogenisator während 5 min. homogenisiert. Man erhält eine weisse Emulsion.

In analoger Weise wird eine Vergleichsemulsion aus den oben angegebenen Bestandteilen der Zusammensetzungen W1 und O1 hergestellt, wobei jedoch in der Zusammensetzung W1 die liposomale SiO₂-Nanopartikeldispersion und das Carboxymethylglucan weggelassen wurden.

### Beispiel 4

Test der Wirksamkeit dieser Zubereitung

In einem klinischen Test an 20 freiwilligen Probanden wurde eine Emulsion gemäß Beispiel 3 und Vergleichsbeispiel 1 auf ihre kosmetischen Eigenschaften überprüft. Die Studie wurde doppelblind randomisiert.

### Studienprofil

Das **hydratatisierende** Potential wurde wie folgt ermittelt:
- Anzahl von freiwilligen Probanden: 10 mit trockener Haut
- Dauer: 3 Stunden ( Messungen T0h, T1h,T2h,T3h)
- Messeinheit: Corneometer PMD 700 Multidiagnostik der Firma Courage und Khazaka
- Untersuchte Stelle: Bein.

| **Statistischer Mittelwert** | **T0h** | **T1h** | **T2h** | **T3h** |
|---|---|---|---|---|
| | | 81,12% | 83,07% | 75,77% |

Im Ergebnis besitzt die Emulsion gemäß Beispiel 3 eine eindrucksvolle hydratisierende Wirkung, welche im statischen Durchschnitt nach 3 Stunden noch 75,77 % erreicht.

### Anti - Falten Wirkung

- 20 freiwillige weibliche Probandinnen im Alter zwischen 45 und 65 (im Schnitt 55,5) Jahren mit mittelschwerer bis starker Faltenbildung - insbesondere auch im Augenbereich.
- Anwendungszeitrahmen :28 Tage
- Anwendungsfrequenz: 2x täglich - früh und abends
- Lokalisation: Gesicht - insbesondere auch Augenpartien (Krähenfüße)
- Studienziel: objektive Überprüfung des Mikroreliefs und Hautfaltenprofils.

Die Untersuchungen wurden durchgeführt in einem Zeitraum von 28 Tagen.

### Methodik

Jede Probandin wurde eingangs und am Ende der Studie dermatologisch untersucht. Nach Abklärung der Ein- und Ausschlusskriterien wurden entsprechend der Doppelblind - Randomisierung die Emulsionen des Beispiels 3 bzw. des Vergleichsbeispiels 1 abgegeben.

Eine Fotodokumentation der Falten wurde am ersten Tag und Tag 28 durchgeführt. Die Probandinnen wurden angewiesen gemäß der Vorgaben Tagesaufzeichnungen zu registrieren, die jeweiligen Emulsionen täglich in der Früh und am Abend anzuwenden, die gewohnten Reinigungsmaßnahmen beizubehalten, exzessive Sonnenbelastungen - Solarium zu meiden und keine weiteren kosmetischen Produkte zu verwenden.

### Ergebnisse

### Köperlotion gemäß Beispiel 3:

Die statistischen Auswertungen der Faltenprofilmessungen und der fotographischen Dokumentationen ergeben im Schnitt eine signifikante 19,82%ige Verringerung der Faltenoberfläche bzw. des Faltenareals, eine um 4,49% Reduktion der Anzahl der Falten, eine 7,39% Verringerung der Faltengesamtlänge und eine mit 7,38% signifikante Reduktion der durchschnittlichen Faltenlänge.

### Emulsion gemäß Vergleichsbeispiel 1:

Kein Nachweis statistisch relevanter Effekte auf das Faltenprofil oder Faltenlänge.

### Straffungseffekt

Überprüfung der biomechanischen Eigenschaften nach 4 wöchiger Anwendung der beiden Emulsionen des Beispiels 3 bzw. des Vergleichsbeispiels 1.
Methode: DTM (Dermal Torque Meter)
Messareal: linke Wangenpartie

### Ergebnisse

### Emulsion gemäß Beispiel 3:

Nach 4 wöchiger Anwendung konnte ein statistisch relevanter Straffungseffekt und eine Verbesserung des Wasserhaushaltes ermittelt werden. Dabei steht der Straffungseffekt im Vordergrund.

### Emulsion gemäß Vergleichsbeispiel 1:

Kein Nachweis statistisch relevanter Effekte auf das Faltenprofil oder Faltenlänge.

### Beispiel 5

### Test der Wirksamkeit dieser pharmazeutischen Zubereitung

In einem klinischen Test an 30 Probanden wurde eine Emulsion gemäß Beispiel 3 und Vergleichsbeispiel 1 auf ihre dermatologischen Eigenschaften überprüft. Die Studie war doppelblind Placebo- kontrolliert und randomisiert.

Indikation: leichte bis mittelschwere Neurodermitis - atopische Dermatitis- nachfolgend DA genannt.

### Studiendesign

- 30 freiwillige Probanden im Alter zwischen 18 und 42 Jahren mit leichter bis mittelschwerer DA nach den Kriterien von Diepgen und Hanflin.
- Anwendungszeitrahmen :40 Tage
- Anwendungsfrequenz: 2x täglich - früh und abends
- Lokalisation: abhängig vom individuellem Krankheitsbild.
- 15 Probanden erhielten die Emulsion gemäß Beispiel 3, 15 Probanden die Emulsion gemäß Vergleichsbeispiel 1.
- Klinische und apparative Auswertung: T0, T20 and T40
- Statistische Analyse: Anova, t-test

### Einschlusskriterien

- Alter über 18 Jahre mit moderater bis mittelschwerer DA nach den Kriterien von Hanifin und Rajka
- Keine Anwendung anderer medizinischer oder kosmetischer Produkte während der Studie

### Ausschlusskriterien

- Patienten mit schwerer exsudativer DA - Sekundärinfektionen
- Patienten mit Cortison - und oder immunmodulatorischer Behandlung
- Keine exzessive natürliche oder künstliche Lichtexposition während der Studie

### Auswertung

subjektive Einschätzung
objektive Beurteilung des Hautzustandes durch den Dermatologen. Der Schweregrad wird beurteilt nach den SCORAD Richtlinien.

| | |
|---|---|
| Kontrolle : | T1 nach der dritten Woche |
| | T2 nach Abschluss |

apparative Analyse:
- Hydration: Corneometer® CM 825
- Barriere Funktion: Tewameter® TM 200
- Hautzustand - weich/ rauh: Visioscan® VC

Der Schweregrad wird ermittelt nach den SCORAD Richtlinien.

### Ergebnisse

Die Auswertung nach Studienabschluss ergibt eine statistisch signifikante, erhebliche Verbesserung des Hautbildes bei 85,3% der Patienten aus der verum Gruppe (Emulsion gemäß Beispiel 3). In vergleichbarem Maße verbessern sich die objektiven Hautparameter wie Hydratation und Barrierefunktion.

Mit der Vergleichsemulsion konnte keine statistisch relevate Verbesserung nachgewiesen werden.

## Patentansprüche

1. Zusammensetzung, die eine liposomale Dispersion von SiO₂-Nanopartikeln und ein β-1,3-verknüpftes Glucanetherderivat umfasst.

2. Zusammensetzung nach Anspruch 1**, dadurch gekennzeichnet, dass** es sich bei dem β-1,3-verknüpften Glucanetherderivat um ein carboxymethyliertes β-1,3-verknüpftes Glucanetherderivat mit einem Carboxymethylierungsgrad von 0,75 handelt.

3. Pharmazeutische Zubereitung, die eine liposomale Dispersion von SiO₂-Nanopartikeln, ein β-1,3-verknüpftes Glucanetherderivat und einen pharmazeutisch akzeptablen Träger enthält.

4. Pharmazeutische Zubereitung nach Anspruch 3, **dadurch gekennzeichnet, dass** die liposomale Dispersion 0,1 - 60 Gew.-% SiO₂, vorzugsweise 0,5 - 20 Gew.-% SiO₂ enthält.

5. Pharmazeutische Zubereitung nach Anspruch 3 oder 4, **dadurch gekennzeichnet, dass** die Partikelgrösse des SiO₂ in einem Bereich von 1 - 40 nm, vorzugsweise 2 - 20 nm liegt.

6. Pharmazeutische Zubereitung nach einem der Ansprüche 3 bis 5, **dadurch gekennzeichnet, dass** die Teilchengrösse der liposomalen Dispersion im Bereich von 20 - 400 nm, vorzugsweise 30 - 100 nm liegt.

7. Pharmazeutische Zubereitung nach einem der Ansprüche 3 bis 6, **dadurch gekennzeichnet, dass** der Gehalt an liposomaler Dispersion 0,1 - 60 %, vorzugsweise 0.5 - 20 % beträgt.

8. Pharmazeutische Zubereitung nach einem der Ansprüche 3 bis 7, **dadurch gekennzeichnet, dass** der Gehalt an dem β-1,3-verknüpften Glucanetherderivat 0,01 - 0,5 Gew.-%, vorzugsweise 0,02 - 0,2 Gew.-% beträgt.

9. Kosmetische Zubereitung, die eine liposomale Dispersion von SiO₂-Nanopartikeln, ein β-1,3-verknüpftes Glucanetherderivat und einen kosmetisch akzeptablen Träger enthält.

10. Kosmetische Zubereitung nach Anspruch 9**, dadurch gekennzeichnet, dass** die liposomale Dispersion 0,1 - 60 Gew.-% SiO₂, vorzugsweise 0,5 - 20 Gew.-% SiO₂ enthält.

11. Kosmetische Zubereitung nach Anspruch 9 oder 10, **dadurch gekennzeichnet, dass** die Partikelgrösse des SiO₂ in einem Bereich von 1 - 40 nm, vorzugsweise 2 - 20 nm liegt.

12. Kosmetische Zubereitung nach einem der Ansprüche 9 bis 11, **dadurch gekennzeichnet, dass** die Teilchengrösse der liposomalen Dispersion im Bereich von 20 - 400 nm, vorzugsweise 30 - 100 nm liegt.

13. Kosmetische Zubereitung nach einem der Ansprüche 9 bis 12, **dadurch gekennzeichnet, dass** der Gehalt an liposomaler Dispersion 0,1 - 60 %, vorzugsweise 0.5 - 20 % beträgt.

14. Kosmetische Zubereitung nach einem der Ansprüche 9 bis 13, **dadurch gekennzeichnet, dass** der Gehalt an dem β-1,3-verknüpften Glucanetherderivat 0,01 - 0,5 Gew.-%, vorzugsweise 0,02 - 0,2 Gew.-% beträgt.

## Claims

1. Composition comprising an SiO₂-nanoparticle liposomal dispersion and a β-1,3-linked glucan ether derivative.

2. Composition according to claim 1, **characterised in that** the β-1,3-linked glucan ether derivative is a carboxymethylated β-1,3-linked glucan ether derivative with a degree of carboxymethylation of 0.75.

3. Pharmaceutical preparation containing an SiO₂-nanoparticle liposomal dispersion, a β-1,3-linked glucan ether derivative and a pharmaceutically acceptable carrier.

4. Pharmaceutical composition according to claim 3, **characterised in that** the liposomal dispersion contains 0.1 to 60 % by weight SiO₂, preferably 0.5 to 20 % by weight SiO₂.

5. Pharmaceutical composition according to either claim 3 or claim 4, **characterised in that** the SiO₂ particle size is between 1 and 40 nm, preferably between 2 and 20 nm.

6. Pharmaceutical composition according to any one of claims 3 to 5, **characterised in that** the particle size of the liposomal dispersion is between 20 and 400 nm, preferably between 30 and 100 nm.

7. Pharmaceutical composition according to any one of claims 3 to 6, **characterised in that** the liposomal dispersion content is between 0.1 and 60 %, preferably between 0.5 and 20%.

8. Pharmaceutical composition according to any one of claims 3 to 7, **characterised in that** the β-1,3-linked glucan ether derivative content is between 0.01 and 0.5 % by weight, preferably between 0.02 and 0.2 % by weight.

9. Cosmetic preparation containing an SiO₂-nanoparticle liposomal dispersion, a β-1,3-linked glucan ether derivative and a cosmetically acceptable carrier.

10. Cosmetic preparation according to claim 9, **characterised in that** the liposomal dispersion contains 0.1 to 60 % by weight SiO₂, preferably 0.5 to 20 % by weight SiO₂.

11. Cosmetic preparation according to either claim 9 or claim 10, **characterised in that** the SiO₂ particle size is between 1 and 40 nm, preferably between 2 and 20 nm.

12. Cosmetic preparation according to any one of claims 9 to 11, **characterised in that** the particle size of the liposomal dispersion is between 20 and 400 nm, preferably between 30 and 100 nm

13. Cosmetic preparation according to any one of claims 9 to 12, **characterised in that** the liposomal dispersion content is between 0.1 and 60 %, preferably between 0.5 and 20 %.

14. Cosmetic preparation according to any one of claims 9 to 13, **characterised in that** the β-1,3-linked glucan ether derivative content is between 0.01 and 0.5 % by weight, preferably between 0.02 and 0.2 % by weight

## Revendications

1. Composition qui comprend une dispersion liposomale de nanoparticules de SiO₂ et un dérivé d'éther de glucane à liaison β-1,3.

2. Composition selon la revendication 1, **caractérisée en ce que** le dérivé d'éther de glucane à liaison β-1,3 est un dérivé d'éther de glucane à liaison β-1,3 carboxyméthylé présentant un degré de carboxyméthylation de 0,75.

3. Préparation pharmaceutique qui contient une dispersion liposomale de nanoparticules de SiO₂, un dérivé d'éther de glucane à liaison β-1,3 et un véhicule pharmaceutiquement acceptable.

4. Préparation pharmaceutique selon la revendication 3, **caractérisée en ce que** la dispersion liposomale contient de 0,1 à 60 % en poids de SiO₂, de préférence, de 0,5 à 20 % en poids de SiO₂.

5. Préparation pharmaceutique selon la revendication 3 ou 4, **caractérisée en ce que** la taille des particules de SiO₂ se situe dans une plage de 1 à 40 nm, de préférence, de 2 à 20 nm.

6. Préparation pharmaceutique selon l'une des revendications 3 à 5, **caractérisée en ce que** la taille des particules de la dispersion liposomale se situe dans une plage de 20 à 400 nm, de préférence de 30 à 100 nm.

7. Préparation pharmaceutique selon l'une des revendications 3 à 6, **caractérisée en ce que** la teneur en dispersion liposomale est de 0,1 à 60 %, de préférence, 0,5 à 20 %.

8. Préparation pharmaceutique selon l'une des revendications 3 à 7, **caractérisée en ce que** la teneur en dérivé d'éther de glucane à liaison β-1,3 est de 0,01 à 0,5 % en poids, de préférence de 0,02 à 02 % en poids.

9. Préparation cosmétique qui contient une dispersion liposomale de nanoparticules de SiO₂, un dérivé d'éther de glucane à liaison β-1,3 et un véhicule cosmétiquement acceptable.

10. Préparation cosmétique selon la revendication 9, **caractérisée en ce que** la dispersion liposomale contient 0,1 à 60 % en poids de SiO₂, de préférence 0,5 à 20 % en poids de SiO₂.

11. Préparation cosmétique selon la revendication 9 ou 10**, caractérisée en ce que** la taille des particules de SiO₂ se situe dans une plage de 1 à 40 nm, de préférence de 2 à 20 nm.

12. Préparation cosmétique selon l'une des revendications 9 à 11, **caractérisée en ce que** la taille des particules de la dispersion liposomale se situe dans une plage de 20 à 400 nm, de préférence de 30 à 100 nm.

13. Préparation cosmétique selon l'une des revendications 9 à 12, **caractérisée en ce que** la teneur en dispersion liposomale est de 0,1 à 60 %, de préférence de 0,5 à 20 %.

14. Préparation cosmétique selon l'une des revendications 9 à 13, **caractérisée en ce que** la teneur en dérivé d'éther de glucane à liaison β-1,3 est de 0,01 à 0,5 % en poids, de préférence de 0,02 à 0,2 % en poids.
